# EUROPEAN PATENT SPECIFICATION

(11) **EP 1 235 519 B1**
(45) Date of publication and mention of the grant of the patent: **02.04.2008**
(21) Application number: 00973331.2
(22) Date of filing: 24.10.2000
(51) Int. Cl.: A61B 10/00

(54) **APPARATUS FOR INTESTINAL SAMPLING AND USE THEREOF**
GERÄT ZUR INTESTINALEN PROBEENTNAHME UND DESSEN VERWENDUNG
DISPOSITIF SERVANT A PRELEVER UN SPECIMEN DE L'INTESTIN ET SON UTILISATION

(30) Priority: 26.10.1999 SE 9903872
(43) Date of publication of application: 04.09.2002
(73) Proprietor: Alimenta Medical AB, 102 49 Stockholm (SE)
(72) Inventor: HÄLLGREN, Roger, S-740 22 Bälinge (SE)
(74) Representative: Aldenbäck, Ulla Christina
(86) International application number: PCT/SE2000/002063
(87) International publication number: WO 2001/030243

(56) References cited:
- EP-A1- 0 219 461
- WO-A1-95/32668
- US-A- 3 877 464
- US-A- 4 735 212

## Description

### Technical field of the invention

The present invention relates to the study of pathological changes in the gastrointestinal system, and more particularly to an apparatus for taking intestinal samples.

### Background of the invention

Among the various techniques that are used today to study pathological changes in the gastro-intestinal system are X-ray contrast examination, computorized tomography and magnetic resonance tomography. Endoscopy, i.e. insertion into the gastrointestinal tract of a stomach or intestinal viewer (gastroscopy, colonoscopy, rectoscopy) makes it possible to inspect the intestinal wall and to take samples from the intestinal mucous membrane (biopsy) which then may be examined by e.g. microscopy. Recently, also intestinal perfusion systems have been used. All these techniques are expensive, time-consuming and more or less trying to the patient. There is therefore a need for a simple methodology that makes it possible to study pathological processes in the intestinal mucous membrane without using biopsy or perfusion.

WO 95/32668 discloses a rectal instrument to be used in allergy diagnosis and designed for insertion into the colonic tract to take samples from the lumen of the large intestine after provocation of the mucous membrane with an allergen. The instrument comprises a tube-like element having a terminal part that is expandable, specifically an inflatable balloon. Subareas on the balloon have a diffusable allergen absorbed or adsorbed thereto, and on the same or separate subareas, a receptor(s) for one or more inflammation markers, usually antibodies specific to the relevant marker(s), are bound to the balloon. The instrument is inserted into the rectum, whereupon the balloon is inflated such that the outer surface of the balloon contacts the mucous membrane. The allergen diffuses to the rectal mucous membrane and, if the individual concerned is allergic to the allergen in question, will cause the release of inflammation markers which in turn diffuse to the lumen where they eventually are captured by the receptor(s) on the balloon surface. After the marker or markers have been released and bound to the balloon, the balloon is deflated and the device is removed and analyzed with respect to the bound marker(s).

The above instrument has, however, the drawback that the areas with diffusible allergen and the areas with immobilized receptor on the balloon surface may be damaged by contact with the internal sphincter when inserted and retracted through the patient's anus. It may also be mentioned, that, generally, immobilizing receptors on the balloon surface could cause problems in that hazardous residues from the coupling of the receptor(s) may remain on the surface.

### Summary of the invention

The present invention aims at overcoming the disadvantages of the prior art devices or instruments and provide an apparatus for rectal insertion for studying pathological processes in the intestinal mucous membrane without the use of biopsy or perfusion. Like the instrument described in WO 95/32668 above, the apparatus of the present invention is based on contacting an expandable part thereof with the intestinal wall for sampling, and optionally also for provocation of the intestine with allergen. According to a basic concept of the invention, however, the sampling area(s) and provocation area(s), when present, of the expandable part essentially are protected from contact with the intestinal wall when the expandable part is inserted and retracted from the colon through the patient's anus.

The present invention therefore provides an apparatus for taking an intestinal sample in a human or animal patient, comprising a holder part and an expandable part supported by the holder part and having at least one sampling area. In a non-expanded state of the expandable part, the expandable part is rectally insertable, and in an expanded state, the sampling area or areas of the expandable part are adapted to contact the intestinal wall. The apparatus is characterized in that it comprises means for protecting the sampling area or areas from contact with the intestinal wall and intestinal fluid when the expandable part in its non-expanded state is rectally inserted into the intestinal tract.

Preferably, the apparatus comprises means for protecting the sampling area or areas also when removing the expandable part from the intestine.

While essentially the whole area of the expandable part could be a sampling area, it is preferred that one or preferably, more subareas thereof are used as sampling areas and provocation areas.

The sampling area or areas on the expandable part may have one or more receptors for, for example, an inflammation or cancer marker or the like bound to the surface thereof (similarly as in the device described in WO 95/32668 above). Such inflammation markers may, for example, be derived from neutrophilic granulocytes, eosinophilic granulocytes, mast cells/basophilic granulocytes, or may be a cytokine, a prostaglandin or a plasma protein, such as albumin.

While the sampling area or areas in this case may have the receptor(s) immobilized directly to the surface of the expandable part, it is preferred that the sampling area or areas comprise a surface element of suitable material attached to the expandable part surface. Such a material may exhibit a two-dimensional surface to which the receptor or receptors are immobilized, or constitute a three-dimensional matrix structure in which the receptor or receptors are immobilized in depth.

Alternatively, and at least in some aspects also preferably, the sampling area or areas comprise absorbing material (without any immobilized receptor) capable of effectively sucking up water and substances dissolved therein (such as proteins, enzymes, hormones etc) when contacted with the intestinal mucosa, such that the content of such substances in the absorbed material may be analyzed. After sampling, a reactant or reactants may be added directly to the absorbing material to detect the presence of a certain substance or substances through a colour reaction or similar. Usually, however, the absorbing material is removed from the expandable part after sampling and the absorbed substances extracted and analyzed separately by various analytical methods, such as electrophoresis, radioimmunological techniques, enzyme immunosorbent assay (ELISA) or nephelometry, just to mention a few.

While it is within the scope of the present invention that the apparatus may be designed for sampling at arbitrary sites along the intestinal tract, it is presently preferred to take samples in the lumen of the large intestine. Such sampling may, for example, advantageously be used for testing for markers for allergy, especially food allergy including e.g. celiac disease, after provoking the colonic mucosa with a desired allergen(s), e.g. gluten (as described in the above-mentioned WO 95/32668).

The expandable part is advantageously an inflatable member of a flexible or elastic material, for example a balloon or an elastic tubular member.

In one embodiment of apparatus according to the present invention, the sampling area or areas on the expandable part are protected by a protective cover until the expandable part has reached the desired section of the intestine to be studied. Prior to or after expanding the expandable part, the cover is removed, e.g. mechanically by pulling means provided on or associated with the apparatus part outside the body, to permit sampling by contacting the sampling area or areas with the intestinal wall.

In another embodiment, the sampling area or areas on the expandable part are protected by a cover which may be opened in connection with the sampling and then be re-closed over the sampling area(s) after the sampling is completed. Such a cover may, for example, be of capsule or shell type, for instance a two-part shell or capsule wherein the two parts together, preferably with some overlap, cover the sampling area(s) in the non-expanded state of the expandable part, but which are removed from and expose the sampling area(s) in the expanded state.

In still another embodiment, the expandable part is displaceably mounted (e.g. telescopically) within the holder part of the apparatus so that the expandable part is kept within the holder part during the rectal insertion and retraction of the apparatus, and brought out of the holder part at the test site to be expanded.

In yet another embodiment, the sampling area or areas on the expandable part are depressed in relation to the remaining surface of the expandable part when the latter is in its non-expanded state to thereby prevent contact with the intestinal wall. If, for example, the expandable part is a balloon or elastic tube or cylinder with sampling elements on the surface, the holder part supporting the balloon or elastic tube may have a recess or recesses for receiving each sampling element when the balloon is deflated.

Like the apparatus described in WO 95/32668, the expandable part may also comprise means for allergen presentation to provoke the intestine before the sampling. Such means may be the surface itself of the expandable part or a special element(s) having the allergen(s) diffusively bound or adsorbed thereto. The allergen-presenting area(s) will, of course, also be protected by the protective means at the same time as the sampling area(s). Optionally, the allergen-presenting area or areas may be the same as the sampling areas.

### Brief description of the drawings

Figure 1A is a schematic perspective and partly sectional view of an embodiment of an apparatus of the present invention with the expandable part of the apparatus in its expanded state and applied in the rectum of a patient.
Figure 1B is a partial view corresponding to that of Fig 1A but with the expandable part of the apparatus in its non-expanded state before insertion into the rectum of a patient.
Figure 1C is a partial view corresponding to that in Figure 1B but showing the expandable part of the apparatus during insertion into the rectum.
Figure 2 is a schematic illustration of separate sampling material member on the expandable part of the apparatus shown in Figure 1A.
Figure 3A is a schematic partial view in section of another embodiment of apparatus of the invention with the expandable apparatus part protected by protective means.
Figure 3B is a corresponding view to that in Figure 3A with the protection means of the expandable part withdrawn.
Figure 4 is a schematic partial view in section of yet another embodiment of apparatus of the invention in a non-expanded state and showing a sampling surface element protected by being received in a recess in the expandable part.

### Detailed description of the invention

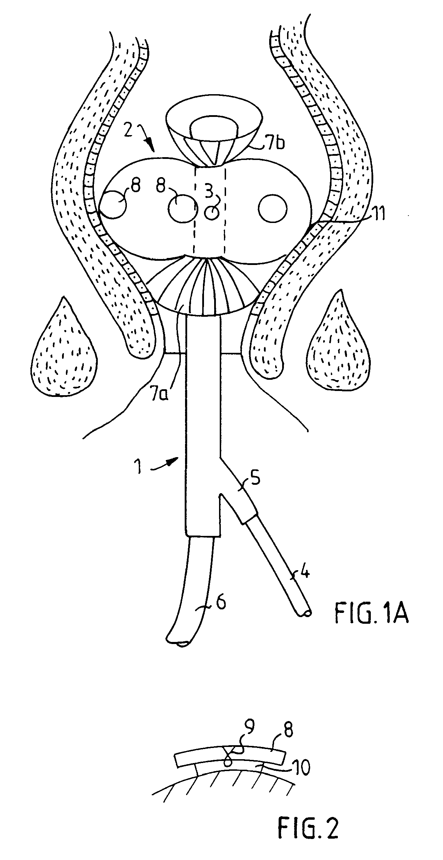

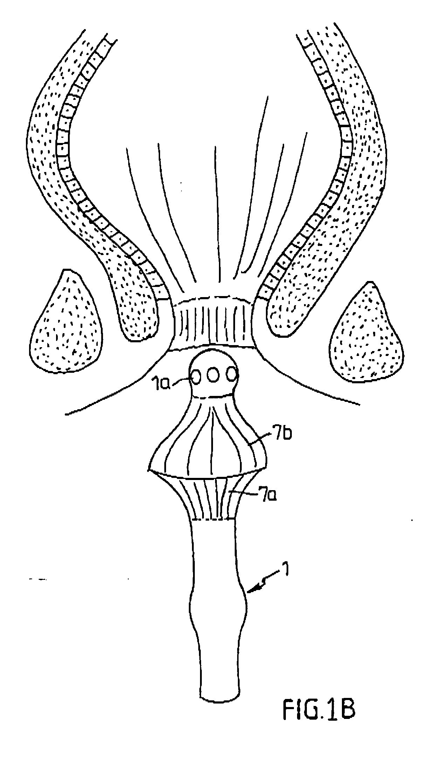

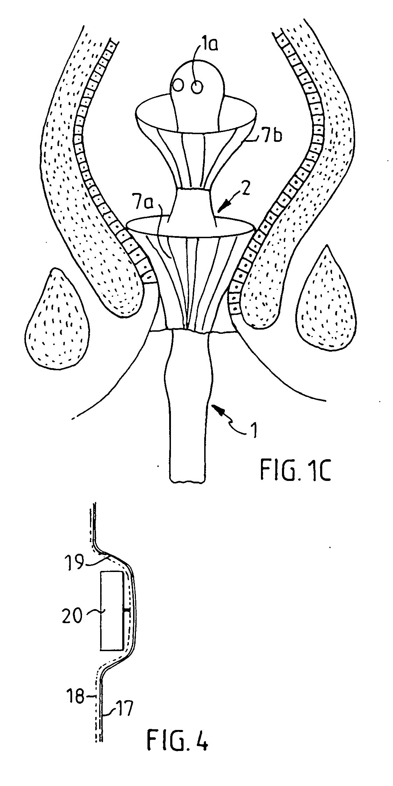

The apparatus illustrated in Figs. 1A to 1C comprises a holder part including a tubular member 1, and at one end of the tubular member 1, an expandable part 2. The latter is typically a balloon or a balloon-like member, for example made of silicone, and extends over an opening 3 (see Fig. 1A) of a channel (not shown) extending through the tubular member 1 and through which air (or other gas or fluid, if desired) may be supplied via a tube 4 attached to a branched tube part 5 of the tubular member 1 to inflate, or expand, the balloon. A through-channel (not shown) extends from the lower end of tubular member 1 to openings 1a (Fig. 1B) at the top end thereof and for permitting pressure equalization between the surroundings and the internal parts of the intestine when the apparatus is used. In the illustrated case, the through-channel is connected to a tube 6 attached to the tubular member 1.

The balloon 2 comprises a protective cover of flexible or resilient material. In the illustrated case, and as is best shown in Fig. 1B, the cover is of a two-part capsule or shell type, e.g. of plastics, such as PVC or silicone, comprising a rear (or lower) shell part 7a and a fore (or upper) shell part 7b. In the deflated state of the balloon shown in Fig. 1B, the fore shell part 7b slightly overlaps the rear shell part 7a such that the two shell parts together totally cover the balloon 2. (The terms "fore" and "rear", respectively, refer to the direction of inserting the instrument into the intestine). The rear shell part 7a is attached to tubular member 1 at the rear end of the shell part, and fore shell part 7b is attached to tubular member 1 at the fore end of the shell part, leaving the remainder of the two shell parts free from the balloon 2. Alternatively, the shell parts 7a, 7b are integral with the balloon 2.

The surface of the balloon 2 (Fig. 1A) has, on a central part thereof, a number of pads or patches 8 of absorptive material, e.g. cellulose, attached to the surface thereof, e.g. three or more patches equidistantly spaced along the balloon periphery. The patches 7 may be attached directly to the balloon 2, or, via another member attached to the balloon surface such as shown by way of example only in Fig. 2. Here, the patch 8 is attached, e.g. by a stitch 9, to a support member 10, e.g. also of cellulose, which in turn is attached to the surface of balloon 2, e.g. by gluing. In this way, the absorptive capability or structure of the patch is essentially unaffected by the attachment to the balloon.

Inflation and deflation, respectively, of the balloon 2 may be achieved by means of, for example, a syringe or a pumping device, such as a flexible or elastic ball or the like (not shown), connected to tube 4 (Fig. 1A). When inflating the balloon 2 from the deflated state shown in Fig. 1B, the fore and rear shell parts 7a, 7b are brought apart to expose the area of the balloon 2 supporting the patches 8 as shown in Fig. 1A. If the material of the shell parts is elastic, the two shell parts may in fact invert as illustrated in Fig. 1A, to expose a major part of the balloon surface.

In use, the apparatus described above may readily be inserted through the anus of a patient to be tested. When in the non-inflated state, the diameter of the balloon is preferably smaller than the diameter of the anus i.e. smaller than about 15-20 mm (but larger than about 5 mm). For rectal sampling, for example, the apparatus is inserted to an appropriate position into the rectum of a patient, with the balloon 2 in the non-inflated state. Preferably, the patches 8 are moistened with water prior to using the apparatus. In Fig. 1A, the fore part of the apparatus with the balloon 2 has been inserted into the rectum beyond the internal sphincter, and the balloon has then been inflated to uncover the patches 8 and make them contact the colonic mucosa such as at 11. When such contact between the patches and mucosa has been maintained for a predetermined time, e.g. 10 to 15 minutes, the balloon is deflated. The deflation itself may cause the protective shell parts 7a, 7b to return to the patch protecting state shown in Fig. 1B where they enclose the patches 8. In the illustrated embodiment, however, the rear shell part 7a is returned to its protecting state by the forces acting upon the shell part when the balloon is retracted and forced through the anus. In both cases, the patches 8 with absorbed mucous fluid will be efficiently protected by the shell part 7a from further contact with the colonic wall and, importantly, when passing the internal sphincter.

After the apparatus has been removed from the patient, the absorbing patches 8 which have been in contact with the colonic mucosa are removed from the balloon 2 and the absorbed contents is analysed, e.g. by extraction of the absorbing patches 8. The extract may then be analyzed by various methods for different substances, primarily proteins, enzymes or hormones. The patches may also be analyzed without extraction by adding directly to the patches, reagents which give a detectable reaction, e.g. a colour reaction, if the substance tested for is present in the pad and may also be a measure of the amount of the substance in question.

If the apparatus is used for allergy tests (such as food allergy, including celiac disease), provocation of the intestine may be effected by a desired food allergen (e.g. gluten) contained in the patches 8. In such a case, the period of contact between the intestinal mucosa and the patches 8 will, of course, be longer, say 15-60 minutes.

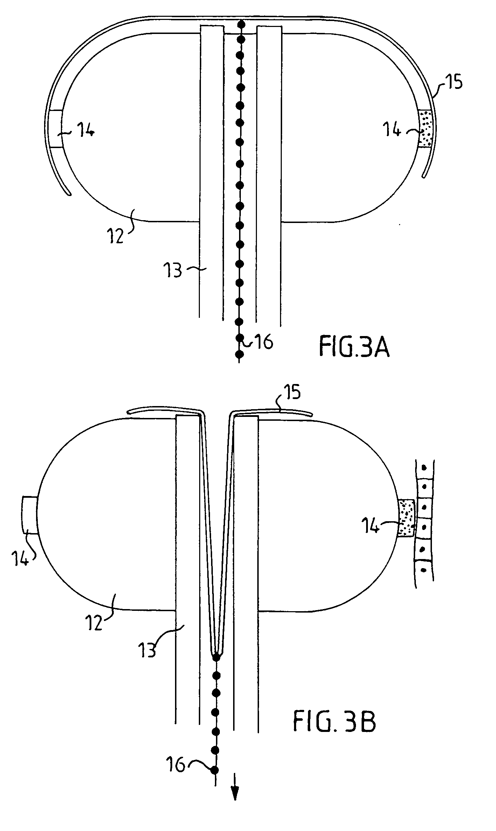

An alternative design of the expandable part of the apparatus of the present invention is shown in Figs. 3A and 3B. Like the embodiment in Figs. 1A to 1C, the expandable part comprises a balloon 12 attached to a tubular support 13 which may be a colonoscope or rectoscope, for example, or a device or instrument specially designed for the present purposes. For clarity, the balloon 12 is shown inflated in Figs. 3A and 3B. A plurality of absorbing elements, such as pads or patches 14 of an absorbing material, are fixed to the balloon surface. A protective cover 15 of flexible material is attached to a pulling means 16, e.g. a rod or, as illustrated, a string extending within the tubular support 13. When pulling the string 16, the protective cover 15 is drawn into the interior of the tubular support 13 to expose the absorbing patches 14 on the balloon surface (Fig. 3B).

In use for taking a rectal sample in a patient to be tested, similarly as described above for embodiment in Figs. 1A to 1C, the fore part of the illustrated apparatus, with the balloon in a deflated state, is inserted into the rectum of the patient to be tested, the cover 15 then efficiently protecting the absorbing patches 14 from contacting the patient's sphincter and the rectal wall. Once inserted to the desired position in the rectum, the balloon 12 is inflated. The cover 15 is then drawn into the tubular support 13 as shown in Fig. 3B, thereby exposing the absorbing patches 14 to make them contact the rectal wall and start absorbing material from the mucous surface thereof. After the predetermined contact time, the balloon 12 is deflated and the apparatus is removed from the patient. The patches 14 are then separated from the balloon and analysed as outlined above.

Fig. 4 illustrates schematically yet an alternative design of the expandable part of the apparatus, where a tubular support 17 for a balloon 18 has a respective recess 19 for each absorbing patch 20. In the deflated state of the balloon 18, the patch 20 is received in the recess 19 with its top surface below the remaining balloon surface as shown in the figure, and thereby protected from contact with the intestinal wall. When the balloon is inflated, it will expand similarly as shown in, for example, Fig. 1A, to contact the patch 20 with the intestinal wall. Upon deflation of the balloon, the patch 20 is sucked into the recess 19 by the negative pressure created in the deflation and is protected when removing the apparatus from the patient. Such recesses for the absorbing patches may, of course, also be used in combination with other apparatus variants of the invention like e.g. those illustrated in Figs. 1A to 1C and Figs. 3A and 3B, respectively.

Analysis of the absorbing material may be performed in various ways. A simple procedure is described below by way of example only.

After the absorbing patches (8, 14) have been removed from the balloon (2, 12), they are immersed in an extraction buffer, e.g. cetyl-N,N,N-trimethylammonium bromide, 0.3% (w/v) in 0.9 % saline. The extraction buffer may, for example, be contained in a syringe with the piston removed, e.g. 2 ml of extraction buffer in a 5 ml volume syringe. After a predetermined time, say, 1 hour, the buffer, including absorbed material contained in the patch or patches, is removed. In the case of a syringe being used, this is accomplished by re-inserting the piston into the syringe and pressing the liquid (including squeezing the patch or patches) into a suitable test container where the expelled liquid is analysed for the presence and, optionally, also quantity, of a desired analyte or desired analytes. Depending on the disease or disorder to be tested for, the analyte may, for example, be an inflammatory mediator, reflecting neutrophilic activity (e.g. myeloperoxidase), eosinophilic activity (ECP = eosinophil cationic protein), or EPO (eosinophil peroxidase or EPX), mast cell /basophilic activity (histamine or tryptase). Further possible analytes are certain cytokines (e.g. IL-6, IL-1, TNF-alfa), prostaglandins (e.g. PGE2).

## Claims

1. An apparatus for taking an intestinal sample of a human or animal patient, said apparatus comprising a holder part (1; 13) and an expandable part (2; 12) supported by the holder part and having one or more sampling areas (8; 14) on the surface thereof, said expandable part (2; 12) in a non-expanded state thereof being rectally insertable into and retractable from the patient's intestine, and in an expanded state, inserted into the patient's intestine, being adapted to contact the intestinal wall with at least one sampling area (8; 14), **characterized in that** the apparatus further comprises protective means (7a, 7b; 15) for preventing said sampling area or areas (8; 14) from contact with the intestinal wall and intestinal fluid at least when said expandable part (2, 12) in the non-expanded state thereof is being rectally inserted into the patient's intestine.

2. The apparatus according to claim 1, **characterized in that** said protective means (7a, 7b) is adapted to prevent said sampling area or areas from contact with the intestinal wall and intestinal fluid also during retraction of said expandable part (2) from the patient's intestine.

3. The apparatus according to claim 1 or 2, **characterized in that** said protective means comprise an at least partially movable protective cover (7a, 7b; 15 is adapted to expose said sampling area or areas (8; 14) in the expanded state of said expandable part (2; 12).

4. The apparatus according to claim 3, **characterized in that** said protective cover comprises two cover parts (7a, 7b) extending lengthwise from opposed end portions of said expandable part (2) to together cover said sampling area or areas (8) at least in the non-expanded state of the expandable part.

5. The apparatus according to claim 4, **characterized in that** said two cover parts (7a, 7b) partially overlap each other at least in the non-expanded state of the expandable part (2).

6. The apparatus according to claim 3, 4 or 5, **characterized in that** said cover or cover parts withdraw from and expose the sampling area or areas when the expandable part is being expanded.

7. The apparatus according to any one of claims 3 to 6, **characterized in that** said protective cover or cover parts (7a, 7b; 15), after having been withdrawn from the sampling area or areas (8, 14) upon expansion of the expandable part, are adapted to returning to the sampling area covering state when the expandable part (2; 12) is being contracted.

8. The apparatus according to any one of claims 4 to 7, **characterized in that** said protective cover is a two-part cover comprising a fore cover part (7b) and a rear cover part (7a), and that the rear cover part (7a), after having been withdrawn from the sampling area or areas (8) upon expansion of the expandable part (2), is adapted to returning to the original sampling area covering state when the expandable part is being retracted from the intestine.

9. The apparatus according to any one of claims 3 to 5, **characterized in that** said protective cover (15) is adapted to uncovering the sampling area or areas (14) in the expanded state of the expandable part (12) through operation of mechanical actuation means (16).

10. The apparatus according to claim 9, **characterized in that** said actuation means comprises a pulling means, such as a string or rod (16).

11. The apparatus according to claim 9 or 10, **characterized in that** said holder part (13) comprises a hollow section into which the cover (15) is adapted to being drawn by said actuation means (16) to thereby uncover said surface element or elements (14).

12. The apparatus according to claim 1 or 2, **characterized in that** said expandable part is movably mounted in said holder part, such that in the non-expanded state, at least the sampling area supporting portion of the expandable part is retracted into the holder part, and in the expanded state, the expandable art projects out of the holder part to expose the sampling area or areas.

13. The apparatus according to claim 12, **characterized in that** said expandable part is mounted in the holder part via telescopic displacement means.

14. The apparatus according to any one of claims 1 to 13, **characterized in that** said protective means comprise a respective recess (19) in the holder part (17) for receiving each sampling area (20) when the expandable part (18) is in its non-expanded state.

15. The apparatus according to any one of claims 1 to 14, **characterized in that** said expandable part comprises an inflatable flexible or elastic member (2; 12).

16. The apparatus according to claim 15, **characterized in that** the expandable part comprises a balloon or elastic tube (2; 12).

17. The apparatus according to any one of claims 1 to 16, **characterized in that** each sampling area comprises a surface element (8; 14) attached to the expandable part (2; 12).

18. The apparatus according to claim 17, **characterized in that** the surface element or elements (8; 14) are capable of absorbing fluid form the intestinal wall when contacted therewith.

19. The apparatus according to any one of claims 1 to 18, **characterized in that** at least one sampling area (8; 14) comprises an immobilized receptor for an analyte in the intestinal fluid.

20. The apparatus according to any one of claims 1 to 19, **characterized in that** at least one sampling area (8; 14) contains a diffusible allergen.

21. The apparatus according to any one of claims 1 to 19, **characterized in that** the expandable part (2; 12) comprises at least one surface area (8; 14) containing diffusible allergen, which surface area is different from the sampling area or areas.

## Patentansprüche

1. Vorrichtung zur Entnahme einer intestinalen Probe aus einem menschlichen oder tierischen Patienten, wobei die Vorrichtung einen Halteteil (1; 13) sowie einen expandierbaren Teil (2; 12) umfasst, der durch den Halteteil abgestützt wird und einen oder mehrere Probenbereiche (8; 14) auf dessen Oberfläche aufweist, wobei der expandierbare Teil (2; 12) in seinem nicht expandierten Zustand rektal in den Darm des Patienten einführbar und aus diesem wieder herausziehbar ist und in einem expandierten Zustand in den Darm des Patienten eingesetzt so angepasst ist, dass er die Darmwand mit zumindest einem Probenbereich (8; 14) berührt, **dadurch gekennzeichnet, dass** die Vorrichtung des Weiteren Schutzelemente (7a, 7b; 15) zur Verhinderung des Kontakts des Probenbereichs oder der Probenbereiche (8; 14) mit der Darmwand und einer Darmflüssigkeit zumindest dann, wenn der expandierbare Teil (2, 12) in seinem nicht expandierten Zustand rektal in den Darm des Patienten eingesetzt ist, umfasst.

2. Vorrichtung gemäß Anspruch 1, **dadurch gekennzeichnet, dass** das Schutzelement (7a, 7b) so angepasst ist, dass es den Kontakt des Probenbereichs oder der Probenbereiche mit der Darmwand und einer Darmflüssigkeit auch während des Wiederherausziehens des expandierbaren Teils (2) aus dem Darm des Patienten verhindert.

3. Vorrichtung gemäß Anspruch 1 oder 2, **dadurch gekennzeichnet, dass** das Schutzelement eine zumindest teilweise bewegliche Schutzabdeckung (7a, 7b; 15) umfasst, die so angepasst ist, dass sie den Probenbereich oder die Probenbereiche (8; 14) im expandierten Zustand des expandierbaren Teils (2; 12) freigibt.

4. Vorrichtung gemäß Anspruch 3, **dadurch gekennzeichnet, dass** die Schutzabdeckung zwei Abdeckungsteile (7a, 7b) umfasst, die sich in Längenrichtung von gegenüberliegenden Endabschnitten des expandierbaren Teils (2) erstrecken, um zusammen den Probenbereich oder die Probenbereiche (8) zumindest im nicht expandierten Zustand des expandierbaren Teils abzudecken.

5. Vorrichtung gemäß Anspruch 4, **dadurch gekennzeichnet, dass** die zwei Abdeckungsteile (7a, 7b) einander zumindest im nicht expandierten Zustand des expandierten Teils (2) teilweise überlappen.

6. Vorrichtung gemäß Anspruch 3, 4 oder 5, **dadurch gekennzeichnet, dass** die Abdeckung oder die Abdeckungsteile sich vom Probenbereich oder den Probenbereichen dann, wenn der expandierbare Teil expandiert wird, zurückziehen und den Probenbereich oder die Probenbereiche freigeben.

7. Vorrichtung gemäß einem der Ansprüche 3 bis 6, **dadurch gekennzeichnet, dass** die Schutzabdeckung oder die Abdeckungsteile (7a, 7b; 15) nach dem Zurückziehen von dem Probenbereich oder den Probenbereichen (8, 14) infolge der Verlängerung des expandierbaren Teils so angepasst sind, dass sie zum Abdeckungszustand für den Probenbereich dann zurückkehren, wenn der expandierbare Teil (2, 12) zusammengezogen wird.

8. Vorrichtung gemäß einem der Ansprüche 4 bis 7, **dadurch gekennzeichnet, dass** die Schutzabdeckung eine zweiteilige Abdeckung ist, die einen vorderen Abdeckungsteil (7b) sowie einen rückseitigen Abdeckungsteil (7a) umfasst, und dass der rückwärtige Abdeckungsteil (7a) nach dem Zurückziehen vom Probenbereich oder den Probenbereichen (8) infolge der Verlängerung des expandierbaren Teils (2) so angepasst ist, dass er zum ursprünglichen Abdeckungszustand für den Probenbereich zurückkehrt, wenn der expandierbare Teil aus dem Darm zurückgezogen wird.

9. Vorrichtung gemäß einem der Ansprüche 3 bis 5, **dadurch gekennzeichnet, dass** die Schutzabdeckung (15) so angepasst ist, dass sie den Probenbereich oder die Probenbereiche (14) im expandierten Zustand des expandierbaren Teils (12) durch den Betrieb mechanischer Betätigungselemente (16) freilegt.

10. Vorrichtung gemäß Anspruch 9, **dadurch gekennzeichnet, dass** das Betätigungselement ein Zugelement wie etwa eine Schnur oder Stange (16) umfasst.

11. Vorrichtung gemäß Anspruch 9 oder 10, **dadurch gekennzeichnet, dass** der Halteteil (13) einen hohlen Abschnitt umfasst, in den die Abdeckung (15) durch das Betätigungselement (16) hineingezogen werden kann, um hierdurch das Oberflächenelement oder die Oberflächenelemente (14) freizulegen.

12. Vorrichtung gemäß Anspruch 1 oder 2, **dadurch gekennzeichnet, dass** der expandierbare Teil beweglich in dem Speicherteil befestigt ist, so dass im nicht expandierten Zustand zumindest der den Probenbereich abstützende Abschnitt des expandierbaren Teils in den Halteteil zurückgezogen ist, und im expandierten Zustand der expandierte Teil aus dem Halteteil hervorsteht, um den Probenbereich oder die Probenbereiche freizugeben.

13. Vorrichtung gemäß Anspruch 12, **dadurch gekennzeichnet, dass** der expandierbare Teil im Halteteil über Teleskop-Verschiebeelemente befestigt ist.

14. Vorrichtung gemäß einem der Ansprüche 1 bis 13, **dadurch gekennzeichnet, dass** das Schutzelement eine jeweilige Aufnahme (19) im Halteteil (17) zum Aufnehmen jedes Probenbereichs (20) umfasst, wenn der expandierbare Teil (18) im nicht expandierten Zustand vorliegt.

15. Vorrichtung gemäß einem der Ansprüche 1 bis 14, **dadurch gekennzeichnet, dass** der expandierbare Teil ein aufblasbares flexibles oder elastisches Element (2; 12) umfasst.

16. Vorrichtung gemäß Anspruch 15, **dadurch gekennzeichnet, dass** der expandierbare Teil einen Ballon oder ein elastisches Rohr (2; 12) umfasst.

17. Vorrichtung gemäß einem der Ansprüche 1 bis 16, **dadurch gekennzeichnet, dass** jeder Probenbereich ein Oberflächenelement (8; 14) umfasst, das am expandierbaren Teil (2; 12) angebracht ist.

18. Vorrichtung gemäß Anspruch 17, **dadurch gekennzeichnet, dass** das Oberflächenelement oder die Oberflächenelemente (8; 14) in der Lage sind, ein Fluid von der Darmwand zu absorbieren, wenn es oder sie mit diesem in Kontakt kommt oder kommen.

19. Vorrichtung gemäß einem der Ansprüche 1 bis 18, **dadurch gekennzeichnet, dass** zumindest ein Probenbereich (8; 14) einen unbeweglichen Rezeptor für ein Analyt im Darmfluid umfasst.

20. Vorrichtung gemäß einem der Ansprüche 1 bis 19, **dadurch gekennzeichnet, dass** zumindest ein Probenbereich (8; 14) ein diffusionsfähiges Allergen enthält.

21. Vorrichtung gemäß einem der Ansprüche 1 bis 19, **dadurch gekennzeichnet, dass** der expandierbare Teil (2; 12) zumindest einen Oberflächenbereich (8; 14) umfasst, der ein diffusionsfähiges Allergen enthält, dessen Oberflächenbereich sich von dem Probenbereich oder den Probenbereichen unterscheidet.

## Revendications

1. Appareil destiné à prélever un échantillon intestinal d'un patient humain ou animal, ledit appareil comprenant une partie de support (1 ; 13) et une partie extensible (2 ; 12) supportée par la partie de support et possédant une ou plusieurs zones d'échantillonnage (8 ; 14) sur la surface de celle-ci, ladite partie extensible (2 ; 12) dans un état non étendu de celle-ci pouvant être insérée par voie rectale dans l'intestin du patient et en être rétractée, et dans un état étendu, est insérée dans l'intestin du patient, étant adaptée pour faire entrer la paroi intestinale en contact avec au moins une zone d'échantillonnage (8 ; 14), **caractérisé en ce que** l'appareil comprend en outre des moyens de protection (7a, 7b ; 15) destinés à empêcher que ladite ou lesdites zones d'échantillonnage (8 ; 14) n'entrent en contact avec la paroi intestinale et le fluide intestinal au moins lorsque ladite partie extensible (2, 12) dans l'état non étendu de celle-ci est insérée par voie rectale dans l'intestin du patient.

2. Appareil selon la revendication 1, **caractérisé en ce que** ledit moyen de protection (7a, 7b) est adapté pour empêcher que ladite ou lesdites zones d'échantillonnage n'entrent en contact avec la paroi intestinale et le fluide intestinal également pendant la rétraction de ladite partie extensible (2) de l'intestin du patient.

3. Appareil selon la revendication 1 ou 2, **caractérisé en ce que** lesdits moyens de protection comprennent un couvercle de protection au moins partiellement mobile (7a, 7b ; 15) adapté pour exposer ladite ou lesdites zones d'échantillonnage (8 ; 14) dans l'état étendu de ladite partie extensible (2 ; 12).

4. Appareil selon la revendication 3, **caractérisé en ce que** ledit couvercle de protection comprend deux parties de couvercle (7a, 7b) s'étendant dans le sens de la longueur depuis des parties d'extrémité opposées de ladite partie extensible (2) pour couvrir ensemble ladite ou lesdites zones d'échantillonnage (8) au moins dans l'état non étendu de la partie extensible.

5. Appareil selon la revendication 4, **caractérisé en ce que** lesdites deux parties de couvercle (7a, 7b) se chevauchent partiellement l'une l'autre au moins dans l'état non étendu de la partie extensible (2).

6. Appareil selon la revendication 3, 4 ou 5, **caractérisé en ce que** ledit couvercle ou lesdites parties de couvercle se retirent de et exposent la zone ou les zones d'échantillonnage lorsque la partie extensible est en train d'être étendue.

7. Appareil selon l'une quelconque des revendications 3 à 6, **caractérisé en ce que** ledit couvercle de protection ou lesdites parties de couvercle (7a, 7b ; 15), après avoir été retirés de la zone ou des zones d'échantillonnage (8, 14) lors de l'extension de la partie extensible, sont adaptés pour revenir à l'état de couverture de la zone d'échantillonnage lorsque la partie extensible (2 ; 12) est en train d'être contractée.

8. Appareil selon l'une quelconque des revendications 4 à 7, **caractérisé en ce que** ledit couvercle de protection est un couvercle en deux parties comprenant une partie de couvercle avant (7b) et une partie de couvercle arrière (7a), et **en ce que** la partie de couvercle arrière (7a), après avoir été retirée de la zone ou des zones d'échantillonnage (8) lors de l'extension de la partie extensible (2), est adaptée pour revenir à l'état de couverture de la zone d'échantillonnage original lorsque la partie extensible est en train d'être rétractée de l'intestin.

9. Appareil selon l'une quelconque des revendications 3 à 5, **caractérisé en ce que** ledit couvercle de protection (15) est adapté pour découvrir la zone ou les zones d'échantillonnage (14) dans l'état étendu de la partie extensible (12) par manoeuvre d'un moyen d'actionnement mécanique (16).

10. Appareil selon la revendication 9, **caractérisé en ce que** ledit moyen d'actionnement comprend un moyen de traction, tel qu'un fil ou une tige (16).

11. Appareil selon la revendication 9 ou 10, **caractérisé en ce que** ladite partie de support (13) comprend une section creuse dans laquelle le couvercle (15) est adapté pour être tiré par ledit moyen d'actionnement (16) pour découvrir de ce fait ledit élément ou lesdits éléments de surface (14).

12. Appareil selon la revendication 1 ou 2, **caractérisé en ce que** ladite partie extensible est montée de manière mobile dans ladite partie de support, de telle sorte que dans l'état non étendu, au moins la portion de support de zone d'échantillonnage de la partie extensible est rétractée dans la partie de support, et dans l'état étendu, la partie extensible fait saillie hors de la partie de support pour exposer la zone ou les zones d'échantillonnage.

13. Appareil selon la revendication 12, **caractérisé en ce que** ladite partie extensible est montée dans la partie de support par le biais de moyens de déplacement télescopique.

14. Appareil selon l'une quelconque des revendications 1 à 13, **caractérisé en ce que** lesdits moyens de protection comprennent un creux respectif (19) dans la partie de support (17) destiné à recevoir chaque zone d'échantillonnage (20) lorsque la partie extensible (18) est dans son état non étendu.

15. Appareil selon l'une quelconque des revendications 1 à 14, **caractérisé en ce que** ladite partie extensible comprend un élément flexible ou élastique gonflable (2 ; 12).

16. Appareil selon la revendication 15, **caractérisé en ce que** la partie extensible comprend un ballonnet ou un tube élastique (2 ; 12).

17. Appareil selon l'une quelconque des revendications 1 à 16, **caractérisé en ce que** chaque zone d'échantillonnage comprend un élément de surface (8 ; 14) fixé à la partie extensible (2 ; 12).

18. Appareil selon la revendication 17, **caractérisé en ce que** l'élément ou les éléments de surface (8 ; 14) sont en mesure d'absorber le fluide provenant de la paroi intestinale lorsqu'ils sont en contact avec celle-ci.

19. Appareil selon l'une quelconque des revendications 1 à 18, **caractérisé en ce que** au moins une zone d'échantillonnage (8 ; 14) comprend un récepteur immobilisé pour une substance à analyser dans le fluide intestinal.

20. Appareil selon l'une quelconque des revendications 1 à 19, **caractérisé en ce que** au moins une zone d'échantillonnage (8 ; 14) contient un allergène diffusible.

21. Appareil selon l'une quelconque des revendications 1 à 19, **caractérisé en ce que** la partie extensible (2 ; 12) comprend au moins une zone de surface (8 ; 14) contenant un allergène diffusible, la zone de surface étant différente de la zone ou des zones d'échantillonnage.
